(19) Europäisches Patentamt
European Patent Office
Office européen des brevets

(11) Veröffentlichungsnummer: **0 262 093**
**A2**

(12) **EUROPÄISCHE PATENTANMELDUNG**

(21) Anmeldenummer: **87810536.0**

(51) Int. Cl.$^4$: **C 07 C 143/66**

(22) Anmeldetag: **17.09.87**

(30) Priorität: **23.09.86 CH 3794/86**

(43) Veröffentlichungstag der Anmeldung:
**30.03.88 Patentblatt 88/13**

(84) Benannte Vertragsstaaten: **CH DE FR GB LI**

(71) Anmelder: **CIBA-GEIGY AG**
**Klybeckstrasse 141**
**CH-4002 Basel (CH)**

(72) Erfinder: **Albrecht, Bernhard**
**Laigweg 32**
**CH-4463 Buus (CH)**

**Beyrich, Jürgen, Dr.**
**Am Tischliweg 3**
**D-7859 Huttingen (DE)**

(54) **Verfahren zur Herstellung von 1-Amino-2-naphthol-4-sulfonsäure.**

(57) Beschrieben wird ein verbessertes halbkontinuierliches und kontinuierliches Verfahren zur Herstellung von 1-Amino-2-naphthol-4-sulfonsäure durch Nitrosierung von 2-Naphthol, Addition von Bisulfit, Sauerstellen mit Mineralsäure und Reduktion das darin besteht, dass man im halbkontinuierlichen Verfahren das Nitrosierungsmittel vorlegt und 2-Naphthol und Mineralsäure in getrennten Strömen simultan zudosiert oder im kontinuierlichen Verfahren die Reaktionsmasse vorlegt und 2-Naphthol, Mineralsäure und das Nitrosierungsmittel in getrennten Strömen simultan zudosiert. Das Produkt kann gegebenenfalls ohne Zwischenisolierung zur Herstellung von Farbstoffen verwendet werden.

EP 0 262 093 A2

**Beschreibung**

Verfahren zur Herstellung von 1-Amino-2-naphthol-4-sulfonsäure

Die Erfindung betrifft ein neues halbkontinuierliches und kontinuierliches Verfahren zur Herstellung von 1-Amino-2-naphthol-4-sulfonsäure durch simultane Zudosierung einer feindispersen wässrigen 2-Naphtholsuspension und Schwefelsäure in getrennten Strömen in ein vorgelegtes Nitrosierungsmittel, bei halbkontinuierlicher Arbeitsweise oder durch simultane Zudosierung von 2-Naphthol, Mineralsäure und das Nitrosierungsmittel in getrennten Strömen bei kontinuierlicher Arbeitsweise in die vorgelegte Reaktionsmasse.

1-Amino-2-naphthol-4-sulfonsäure ist ein wichtiges Zwischenprodukt zur Herstellung von Farbstoffen, vor allem sauren Metallkomplexfarbstoffen. So sind auch viele Verfahren bekannt, dieses Produkt herzustellen. Verwiesen sei beispielsweise auf Organic Synthesis, Vol. I (1941) S. 411 und Vol. II, (1943) S. 42. Gemäss dieser Vorschrift wird die Umsetzung von 2-Naphthol mit Natriumnitritlösung in einer verdünnten wässrigen Lösung durchgeführt und die isolierte Paste des nitrosierten 2-Naphthols mit der 2,7-fachen molaren Menge Natriumhydrogensulfit umgesetzt. Anschliessend wird mit Schwefelsäure angesäuert und das Produkt isoliert. Gemäss BIOS Final Report No. 986, Pt. 1 Item No. 22, Seiten 44-45 und Seite 146-147 wird 1-Amino-2-naphthol-4-sulfonsäure in wässriger Lösung hergestellt.

Bei diesem Verfahren entstehen aber grosse Mengen Natriumsulfat, welches eine Abwasserbelastung darstellt. All diese Verfahren haben zudem den Nachteil, dass sie in sehr verdünnten Lösungen durchgeführt werden müssen und grosse Mengen Natriumhydrogensulfit erforderlich sind.

Aus der DE-A-34 31 695 ist schliesslich ein Verfahren zur Herstellung von 1-Amino-2-naphthol-4-sulfonsäure bekannt durch Nitrosierung von 2-Naphthol, Addition von Bisulfit und Sauerstellung mit Mineralsäure, indem man die Nitrosierung in Gegenwart wassermischbarer organischer Lösungsmittel durchführt. Neben einer geringen Raum-Zeit-Ausbeute bietet dieses Verfahren wegen der Abwasserverschmutzung mit organischen Lösungsmitteln einen schwerwiegenden Nachteil.

Gegenstand der vorliegenden Erfindung ist nun ein Verfahren zur Herstellung von 1-Amino-2-naphthol-4-sulfonsäure, das die genannten Nachteile überwindet. Es beruht auf der Nitrosierung von 2-Naphthol, Addition von Bisulfit, Sauerstellung mit Mineralsäure und Reduktion, wobei man in einem wässrigen Medium arbeitet, und entweder a) im halbkontinuierlichen Verfahren das Nitrosierungsmittel vorlegt und 2-Naphthol und Mineralsäure in getrennten Strömen simultan zudosiert, oder b) im kontinuierlichen Verfahren die Reaktionsmasse vorlegt und 2-Naphthol, Mineralsäure und das Nitrosierungsmittel in getrennten Strömen simultan zudosiert.

Die so erhaltene 1-Amino-2-naphthol-4-sulfonsäure kann direkt, d.h. ohne Zwischenisolierung, zu Farbstoffen wie z.B. Eriochromschwarz weiterverarbeitet werden.

Die genannten Ausgangsverbindungen für dieses Verfahren und ihre Herstellung sind bekannt.

Das als Ausgangsverbindung verwendete 2-Naphthol wird zweckmässigerweise in Form einer feindispersen wässrigen Suspension oder Anschlämmung dem vorgelegten Nitrosierungsmittel zudosiert. Auf Grund der geringen Löslichkeit von 2-Naphthol in der Mineralsäure erweist es sich als vorteilhaft, dieses mittels Kristallisation und Nassmahlung aus einer wässrigen Emulsion in eine feindisperse Form zu überführen. Durch diese Massnahme erreicht man eine deutliche Erhöhung der Reaktionsgeschwindigkeit. Zur Nassmahlung verwendet man die üblichen Mahlaggregate. Um zu einer stabilen Dispersion zu gelangen, kann man die Mahlung in Gegenwart eines Dispergiermittels z.B. Sulfitablauge Netzöl® oder Türkischrotöl sowie eines organischen mit Wasser mischbaren Lösungsmittels beispielsweise Alkohol, durchführen.

Das Verfahren wird vorteilhafterweise so durchgeführt, dass man 2-Naphthol und Mineralsäure in einem solchen Verhältnis zudosiert, dass auf 1 Mol 2-Naphthol 1 bis 1,1 Mol Mineralsäure kommen.

Als Nitrosierungsmittel werden vorzugsweise Alkali- oder Erdalkalinitrite sowie auch organische Nitrite wie Glykolnitrite verwendet. Vorzugsweise wird Natriumnitrit eingesetzt.

Als Mineralsäuren werden vorzugsweise Halogenwasserstoffsäuren, wie Salzsäure, Phosphorsäure, Phosphonsäure, phosphorige Säure sowie insbesondere Schwefelsäure eingesetzt. Es handelt sich dabei in der Regel um eine 5 bis 95 Gew.%ige, insbesondere um eine 25 bis 50 Gew.%ige Mineralsäure.

Als die zur Anlagerung an das Nitrosonaphthol befähigten Bisulfite werden beispielsweise Alkali-, Erdalkali- oder Ammoniumhydrogensulfite, vorzugsweise Natriumhydrogensulfit eingesetzt.

Das halbkontinuierliche Verfahren wird z.B. in der Weise durchgeführt, dass man 2-Naphthol als wässrige, feindisperse Suspension, deren Partikelgrösse vorzugsweise kleiner als 50 µm, insbesondere 1 bis 10 µm ist, simultan in getrennten Strömen mit Mineralsäure, vorzugsweise 35%iger wässriger Schwefelsäure einem Gemisch zudosiert, das wässrige Natriumnitritlösung, gegebenenfalls Restreaktionsmasse, ein 2-Nitrosonaphthol aus der vorangegangenen Reaktion und Eis enthält, und bei einer Reaktionstemperatur von 0 bis 10°, insbesondere 0 bis 5° C, das 2-Naphthol zum 1-Nitroso-2-naphthol umsetzt. Nach dem Ausrühren wird die Reaktionsmasse mit Natriumhydrogensulfit zu 1-Hydroxylamino-2-tetralon-sulfonsäure bei einer Temperatur von 15 bis 30° C umgesetzt und dann zwischen 25 und 90° C im sauren pH-Bereich, beispielsweise 5 bis 5,5, zu 1-Amino-2-naphthol-4-sulfonsäure reduziert. Ist die Isolierung dieses Produkts erwünscht, so erfolgt diese beispielsweise durch Filtration, Waschen und Trocknen, z.B. im Vakuumtrockenschrank.

Die Reduktion der Hydroxylamino-tetralonverbindung wird vorzugsweise in Gegenwart eines Katalysators wie z.B. einem Uebergangsmetallsulfat ($CuSO_4$, $FeSO_4$) durchgeführt. Das Uebergangsmetallsulfat kann wasserhaltig sein.

Das Verfahren kann sowohl halbkontinuierlich oder kontinuierlich durchgeführt werden, wobei es sich als Eintopfverfahren über alle Stufen ausüben lässt.

Das Verfahren kann z.B. durch folgendes Schema veranschaulicht werden:

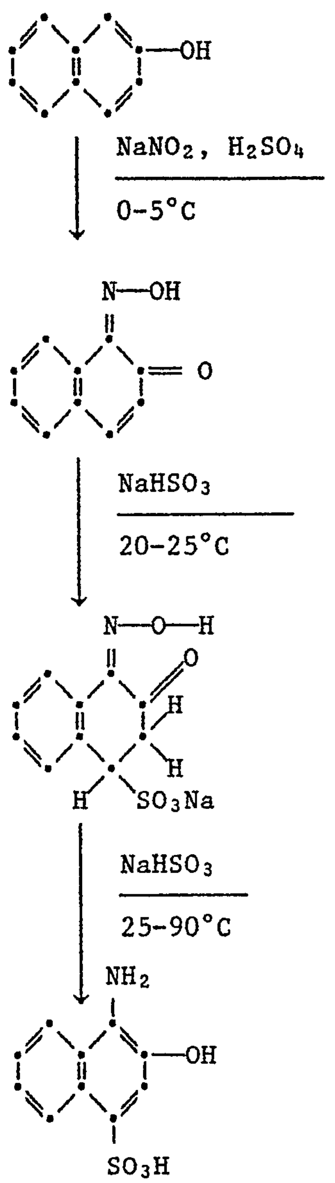


2-Naphthol

Nitrosierung

1-Nitroso-2-naphthol

Bisulfitanlagerung

1-Hydroxylamino-2-tetralon-4-sulfonsäure

Reduktion

1-Amino-2-naphthol-4-sulfonsäure

Ein weiterer Vorteil der erfindungsgemässen Verfahrensweise liegt darin, dass die 1-Amino-2-naphthol-4-sulfonsäure in hohen Ausbeuten und hoher Reinheit erhalten wird, da das 2-Naphthol sehr rasch und vollständig nitrosiert wird. Dadurch kommt es zu keiner örtlichen Anhäufung von Mineralsäure und Freisetzung von nitrosen Gasen im Reaktionsgemisch, wodurch die Bildung von Nebenprodukten weitgehend vermieden wird. Die Konzentration der Mineralsäure bleibt also während der Reaktion im wesentlichen auf tiefem Niveau konstant.

Das folgende Beispiel veranschaulicht die Erfindung. Teile (T) bedeuten Gewichtsteile.

Beispiel 1:

a) Herstellung der 2-Naphtholsuspension-Emulsion und Mahlung

In eine Kugelmühle wird
250 T Wasser,
4 T Sulfitablaugepulver als Dispergiermittel und
144 T 2-Naphthol eingetragen und zusammen fein vermahlen.
Die erhaltene flüssige Suspension wird mit Wasser auf 462 T gestellt.

b) Nitrosierung von 2 Naphthol

In einen Reaktor werden unter Kühlung
500 T Eis und
72,9 T Natriumnitrit gegeben.
Innert 60 Min. werden dann simultan
462 T 2-Naphthol-Emulsion gemäss a) und
147 T Schwefelsäure 35 % getrennt zudosiert.
Das 1-Nitroso-2-naphthol fällt dabei grobkristallin aus; man lässt die Reaktionsmasse bei 5-10°C während einer Stunde ausrühren.

c) Bisulfitanlagerung

Die gemäss b) erhaltene Reaktionsmasse wird mit 572 T wässriger Natriumbisulfit Lösung (40 %) versetzt und die Reaktionsmasse auf 25°C erwärmt.

d) Reduktion

In die Reaktionsmasse lässt man
613 T Schwefelsäure 35 %
1,15 T $CuSO_4 \cdot 5\ H_2O$ bei 25°C zulaufen.
Nach dem Zulauf heizt man die Reaktionsmasse innert 1 Stunde auf ca. 85°C auf.
Die 1-Amino-2-naphthol-4-sulfonsäure beginnt auszufallen.
Nachreaktion 30 Minuten bei 85°C.

e) Filtration

Die dickflüssige Suspension der 1-Amino-2-naphthol-4-sulfonsäure wird bei 85°C auf einer Nutsche abgesaugt und der Filterkuchen mit Wasser in mehreren Portionen gewaschen und anschliessend das Wasser abgesaugt. Anschliessend wird das feuchte Nutschgut bei 80°C im Vakuumtrockenschrank getrocknet. Man erhält
202,6 T 1-Amino-2-naphthol-4-sulfonsäure als weisses Pulver.
Dies entspricht einer Ausbeute von 85 % (bezogen auf eingesetztes Naphthol).

Beispiel 2: Kontinuierliche Nitrosierung

In einem Reaktor mit Mantelkühlung werden
462 T/h 2-Naphtholsuspension (gemäss a) in Beispiel 1 hergestellt)
147 T/h Schwefelsäure (35 %) und
182 T/h Natriumnitritlösung (40 %) in getrennten Strömen zu ausreagierter Reaktionsmasse (aus dem vorhergehendem Ansatz) zudosiert und bei einer Temperatur von 0 bis 5°C und einem pH-Wert von 2-3 umgesetzt. Nach Unterbruch der Speisung lässt man noch 30 Minuten reagieren und entnimmt 60 % der Reaktionsmasse zur Weiterverarbeitung gemäss Stufe c) bis e) nach Beispiel 1.

Mit der verbleibenden Reaktionsmasse kann die semikontinuierliche Nitrosierung wiederholt werden.

**Patentansprüche**

1. Verfahren zur halbkontinuierlichen oder kontinuierlichen Herstellung von 1-Amino-2-naphthol-4-sulfonsäure durch Nitrosierung von 2-Naphthol, Addition von Bisulfit, sauerstellen mit Mineralsäure und Reduktion, dadurch gekennzeichnet, dass man in einem wässrigen Medium arbeitet, und entweder a) im halbkontinuierlichen Verfahren das Nitrosierungsmittel vorlegt und 2-Naphthol und Mineralsäure in getrennten Strömen simultan zudosiert, oder b) im kontinuierlichen Verfahren die Reaktionsmasse vorlegt und 2-Naphthol, Mineralsäure und das Nitrosierungsmittel in getrennten Strömen simultan zudosiert.

2. Verfahren gemäss Anspruch 1a, dadurch gekennzeichnet, dass man in ein wässriges Nitrosierungsmedium eine wässrige Suspension von 2-Naphthol und Mineralsäure in getrennten Strömen simultan zudosiert.

3. Verfahren gemäss Anspruch 1b, dadurch gekennzeichnet, dass man in eine wässrige Reaktionsmasse eine wässrige Suspension von 2-Naphthol, Mineralsäure und Nitrosierungsmittel in getrennten Strömen simultan dosiert.

4. Verfahren gemäss Anspruch 1, dadurch gekennzeichnet, dass man 2-Naphthol und Mineralsäure in einem solchen Verhältnis zudosiert, dass auf 1 Mol 2-Naphthol 1 bis 1,1 Mol Mineralsäure kommen.

5. Verfahren gemäss Anspruch 1, dadurch gekennzeichnet, dass als Mineralsäure Schwefelsäure verwendet wird.

6. Verfahren gemäss Anspruch 2, dadurch gekennzeichnet, dass die Partikelgrösse der wässrigen 2-Naphtholsuspension kleiner als 50 µm ist.

7. Verfahren gemäss Anspruch 1, dadurch gekennzeichnet, dass man die Nitrosierung bei einer Temperatur von 0 bis 10° durchführt.

8. Verfahren gemäss Anspruch 7, dadurch gekennzeichnet, dass man die Nitrosierung bei einer Temperatur von 0 bis 5° C durchführt.

9. Verfahren gemäss Anspruch 1, dadurch gekennzeichnet, dass man die Bisulfitanlagerung bei einer Temperatur von 15 bis 30° C durchführt.

10. Verfahren gemäss Anspruch 1, dadurch gekennzeichnet, dass man die Reduktion bei einer Temperatur von 25 bis 90° C durchführt.

11. Verfahren gemäss Anspruch 1, dadurch gekennzeichnet, dass die Mineralsäurekonzentration während der Reaktion im wesentlichen konstant bleibt.

12. Die nach dem Verfahren gemäss Anspruch 1 erhaltene 1-Amino-2-naphthol-4-sulfonsäure.